(19) **Europäisches Patentamt / European Patent Office / Office européen des brevets**

(11) **EP 1 932 203 B1**

(12) **EUROPEAN PATENT SPECIFICATION**

(45) Date of publication and mention
of the grant of the patent:
**22.04.2009 Bulletin 2009/17**

(51) Int Cl.:
*H01M 10/40* (2006.01)    *H01M 4/02* (2006.01)
*H01M 4/58* (2006.01)

(21) Application number: **06815503.5**

(22) Date of filing: **26.09.2006**

(86) International application number:
**PCT/US2006/037551**

(87) International publication number:
**WO 2007/041113 (12.04.2007 Gazette 2007/15)**

(54) **LITHIUM-ION BATTERY**

LITHIUMIONEN-BATTERIE

BATTERIE AU LITHIUM

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.10.2005 US 243734**

(43) Date of publication of application:
**18.06.2008 Bulletin 2008/25**

(73) Proprietor: **MEDTRONIC, INC.**
**Minneapolis MN 55432-5604 (US)**

(72) Inventors:
• **SCOTT, Erik, R.**
**Maple Grove, MN 55311 (US)**
• **HOWARD, William, G.**
**Roseville, MN 55113 (US)**

(74) Representative: **UEXKÜLL & STOLBERG**
**Patentanwälte**
**Beselerstrasse 4**
**22607 Hamburg (DE)**

(56) References cited:
EP-A1- 0 856 901          WO-A-20/06101779
JP-A- 2005 005 115       US-A1- 2006 210 883
US-B2- 6 589 697

• CONTESTABILE M ET AL: "A comparative study on the effect of electrolyte/additives on the performance of ICP383562 Li-ion polymer (soft-pack) cells" JOURNAL OF POWER SOURCES, ELSEVIER, AMSTERDAM, NL, vol. 119-121, 1 June 2003 (2003-06-01), pages 943-947, XP004430304 ISSN: 0378-7753
• MARKOVSKY B ET AL: "The study of capacity fading processes of Li-ion batteries: major factors that play a role" JOURNAL OF POWER SOURCES, ELSEVIER, AMSTERDAM, NL, vol. 119-121, 1 June 2003 (2003-06-01), pages 504-510, XP004430222 ISSN: 0378-7753
• AURBACH D ET AL: "On the capacity fading of LiCoO2 intercalation electrodes: - the effect of cycling, storage, temperature, and surface film forming additives" ELECTROCHIMICA ACTA, ELSEVIER SCIENCE PUBLISHERS, BARKING, GB, vol. 47, no. 27, 15 October 2002 (2002-10-15), pages 4291-4306, XP004391765 ISSN: 0013-4686

**Description**

BACKGROUND

The present invention relates generally to the field of lithium batteries. Specifically, the present invention relates to lithium-ion batteries that are less susceptible to capacity fade during the life of the batteries.

Lithium-ion batteries include a positive current collector (e.g., aluminum such as_ an aluminum foil) having an active material provided thereon (e.g., $LiCoO_2$) and a negative current collector (e.g., copper such as a copper foil) having an active material (e.g., a carbonaceous material such as graphite) provided thereon. Together the positive current collector and the active material provided thereon are referred to as a positive electrode, while the negative current collector and the active material provided thereon are referred to as a negative electrode.

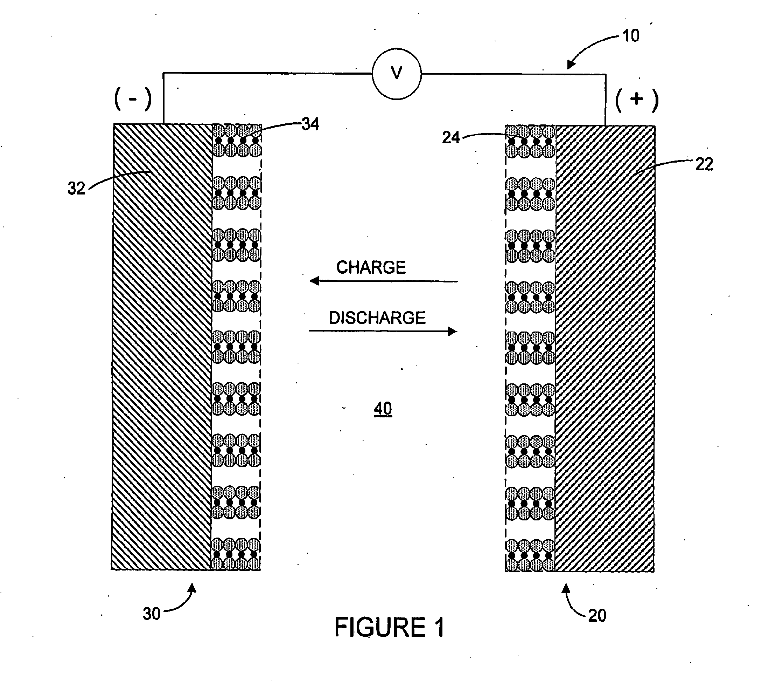

FIGURE 1 shows a schematic representation of a portion of a conventional lithium-ion battery 10 such as that described above. The battery 10 includes a positive electrode 20 that includes a positive current collector 22 and a positive active material 24, a negative electrode 30 that includes a negative current collector 32 and a negative active material 34, an electrolyte material 40, and a separator (e.g., a polymeric microporous separator, not shown) provided intermediate or between the positive electrode 20 and the negative electrode 30. The electrodes 20, 30 may be provided as relatively flat or planar plates or may be wrapped or wound in a spiral or other configuration (e.g., an oval configuration). The electrode may also be provided in a folded configuration.

During charging and discharging of the battery 10, lithium ions move between the positive electrode 20 and the negative electrode 30. For example, when the battery 10 is discharged, lithium ions flow from the negative electrode 30 to the to the positive electrode 20. In contrast, when the battery 10 is charged, lithium ions flow from the positive electrode 20 to the negative electrode 30.

One difficulty with conventional lithium-ion batteries is that they may exhibit a capacity fade over the life of the batteries as a result of cell aging and cycling. As used herein, the term "capacity fade" refers to a loss in battery capacity over the life of the battery.

The medical device industry produces a wide variety of electronic and mechanical devices for treating patient medical conditions. Depending upon the medical condition, medical devices can be surgically implanted or connected externally to the patient receiving treatment. Clinicians use medical devices alone or in combination with drug therapies and surgery to treat patient medical conditions. For some medical conditions, medical devices provide the best, and sometimes the only, therapy to restore an individual to a more healthful condition and a fuller life.

It may be desirable to provide an improved source of battery power for battery powered devices such as medical devices, including implantable medical devices. In such cases, it may be advantageous to provide a battery that may be recharged. It may also be advantageous to provide a battery that may be relatively resistant to capacity fade over the life of the battery. It would also be advantageous to provide a medical device (e.g., an implantable medical device) that utilizes a battery that includes any one or more of these or other advantageous features.

SUMMARY

An exemplary embodiment of the invention relates to a lithium-ion battery that includes a positive electrode, a negative electrode comprising carbon, and an electrolyte containing a first and second additive. The first additive includes a borane or borate compound that acts as an ion receptor and the second additive includes an alkene capable of reacting on a surface of the negative electrode to form an ionically conductive layer.

Another exemplary embodiment of the invention relates to a lithium-ion battery that includes a positive electrode, a negative electrode comprising carbon fiber or a mixture of graphitized mesocarbon microbeads and coke, and an electrolyte comprising a first additive comprising a borane or borate compound that acts as an ion receptor and a second additive comprising an alkene capable of reacting on a surface of the negative electrode to form an ionically conductive layer.

Another exemplary embodiment of the invention relates to a lithium-ion battery that includes a positive electrode, a negative electrode comprising an active material comprising graphitized mesocarbon microbeads and coke, and an electrolyte comprising vinylene carbonate and trimethoxyboroxine additives.

Another exemplary embodiment of the invention relates to a medical device that includes a lithium-ion battery for providing power to the medical device. The lithium-ion battery includes a positive electrode, a negative electrode comprising carbon, and an electrolyte containing a first and second additive. The first additive includes a borane or borate compound that acts as an ion receptor and the second additive includes an alkene capable of reacting on a surface of the negative electrode to form an ionically conductive layer.

BRIEF DESCRIPTION OF THE DRAWINGS

FIGURE 1 is a schematic cross-sectional view of a conventional lithium-ion battery.

FIGURE 2 is a schematic cross-sectional view of a portion of a lithium-ion battery according to an exemplary embodiment.

FIGURE 3 is a graph showing the capacity fade for lithium batteries having a negative electrode comprising MCMB and utilizing various electrolyte additives.

FIGURE 4 is a graph showing the capacity fade for lithium batteries having a negative electrode comprising MCMB and coke and utilizing various electrolyte additives.

FIGURE 5 is a graph showing the capacity fade for lithium batteries having a negative electrode comprising carbon fiber and utilizing various electrolyte additives.

FIGURE 6 is a schematic view of a system in the form of an implantable medical device implanted within a body or torso of a patient.

FIGURE 7 is schematic view of another system in the form of an implantable medical device.

DETAILED DESCRIPTION OF EXEMPLARY EMBODIMENTS

According to an exemplary embodiment, a battery may be provided that may exhibit enhanced resistance to capacity fade over the lifetime of the battery. The battery includes a carbonaceous negative electrode and an electrolyte that includes one or more additives which are intended to reduce the amount of capacity fade experienced by the battery during use. Such a battery may be utilized in any of a wide variety of applications that require relatively consistent capacity over the lifetime of use, including implantable medical devices.

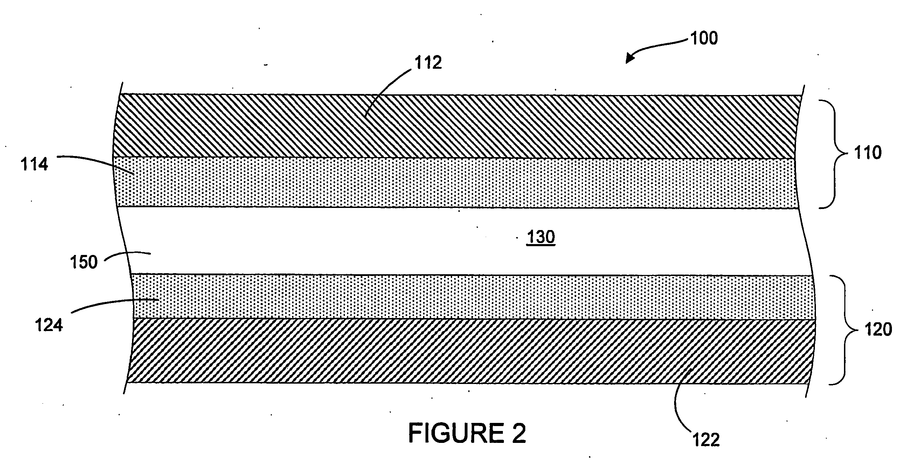

With reference to FIGURE 2, a schematic cross-sectional view of a portion of a lithium-ion battery 100 is shown according to an exemplary embodiment. According to an exemplary embodiment, the battery 100 has a rating of between approximately 10 and 1000 milliampere hours (mAh). According to another exemplary embodiment, the battery has a rating of between approximately 100 and 400 mAh. According to another exemplary embodiment, the battery is an approximately 300 mAh battery. According to another exemplary embodiment, the battery is an approximately 75 mAh battery.

The battery case (not shown) may be made of stainless steel or another metal. According to an exemplary embodiment, the battery case may be made of titanium, aluminum, or alloys thereof. According to another exemplary embodiment, the battery case may be made of a plastic material or a plastic-foil laminate material (e.g., an aluminum foil provided intermediate a polyolefin layer and a polyester layer).

The dimensions of the battery 100 may differ according to a variety of exemplary embodiments. For example, according to one exemplary embodiment in which the electrodes are wound such that they may be provided in a relatively prismatic battery case, the battery has dimensions of between approximately 30-40 mm by between approximately 20-30 mm by between approximately 5-7 mm. According to another exemplary embodiment, the dimensions of the battery are approximately 20 mm by 20 mm by 3 mm. According to another exemplary embodiment, a battery may be provided in the form of a button cell type battery having a diameter of approximately 30 mm and a thickness of approximately 3 mm. It will be appreciated by those of skill in the art that such dimensions and configurations as are described herein are illustrative only, and that batteries in a wide variety of sizes, shapes, and configurations may be produced in accordance with the novel concepts described herein.

The battery 100 includes at least one positive electrode 110 and at least one negative electrode 120. A separator 150 is provided intermediate or between the positive electrode 110 and the negative electrode 120. According to an exemplary embodiment, the separator 150 is a polymeric material such as a polypropylene/polyethelene copolymer or another polyolefin multilayer laminate that includes micropores formed therein to allow electrolyte and lithium ions to flow from one side of the separator to the other. The thickness of the separator 150 is between approximately 10 micrometers ($\mu$m) and 50 $\mu$m according to an exemplary embodiment. According to a particular exemplary embodiment, the thickness of the separator is approximately 25 $\mu$m and the average pore size of the separator is between approximately 0.02 $\mu$m and 0.1 $\mu$m.

According to an exemplary embodiment, the battery 100 is provided as a spirally wound prismatic battery in which cells are hermetically sealed in a stainless steel can with a glass feedthrough. According to other exemplary embodiments, the electrodes may be provided as flat or planar components of the battery 100 or may be provided in a folded configuration. According to one particular exemplary embodiment, the electrodes may be wrapped around a relatively rectangular mandrel such that they form an oval wound coil for insertion into a relatively prismatic battery case. According to other exemplary embodiments, the battery may be provided as a button cell battery, a thin film solid state battery, or as another lithium-ion battery configuration.

According to an exemplary embodiment, the negative electrode is coupled to a stainless steel case by a member or tab comprising nickel or a nickel alloy. An aluminum or aluminum alloy member or tab may be coupled or attached to the positive electrode. The nickel and aluminum tabs may serve as terminals for the battery according to an exemplary embodiment.

The positive electrode 110 includes a current collector 112 made of a conductive material such as a metal. According to an exemplary embodiment, the current collector 112 comprises aluminum or an aluminum alloy and has a thickness between approximately 5 $\mu$m and 75 $\mu$m. According to a particular exemplary embodiment, the thickness of the current collector 112 is approximately 20 $\mu$m. It should also be noted that while the positive current collector 112 has been illustrated and described as being a thin foil material, the positive current collector may have any of a variety of other configurations according to various exemplary embodiments. For example, the positive current collector may be a grid such as a mesh grid, an expanded metal grid, a photochemically etched grid, or the like.

The current collector 112 has a layer 114 of active material provided thereon (e.g., coated on the current collector). While FIGURE 2 shows that the layer 114 is provided on only one side of the current collector 112, it should be understood that a layer of active material similar or identical to that shown as layer 114 may be provided or coated on both sides of the current collector 112.

According to an exemplary embodiment, the layer 114 is a material or compound that includes lithium that may be doped and undoped during discharging and charging of the battery, respectively. According to a particular exemplary embodiment, the layer 114 includes lithium cobalt oxide ($LiCoO_2$). As will be appreciated by those reviewing this document, the positive active material layer may be made of or include various other materials in place of or in addition to $LiCoO_2$ according to various other exemplary embodiments. For example, the positive active material may have a composition that includes any of the various combinations of materials described in U.S. Patent Application No. 10/979,041 titled "Lithium-Ion Battery" and filed on October 29, 2004, the entire disclosure of which is incorporated by reference herein.

According to an exemplary embodiment, the thickness of the layer of active material 114 is between approximately 0.1 $\mu$m and 3 mm. According to another exemplary embodiment, the thickness of the layer of active material 114 is between approximately 25 $\mu$m and 300 $\mu$m. According to a particular exemplary embodiment, the thickness of the layer of active material 114 is approximately 75 $\mu$m.

The negative current collector 122 included as part of the negative electrode 120 is made of a conductive material such as a metal. According to an exemplary embodiment, the current collector 122 is copper or a copper alloy. According to another exemplary embodiment, the current collector 122 is titanium or a titanium alloy. According to another exemplary embodiment, the current collector 122 is nickel or a nickel alloy. It should also be noted that while the negative current collector 122 has been illustrated and described as being a thin foil material, the positive current collector may have any of a variety of other configurations according to various exemplary embodiments. For example, the positive current collector may be a grid such as a mesh grid, an expanded metal grid, a photochemically etched grid, or the like.

According to an exemplary embodiment, the thickness of the current collector 122 is between approximately 100 nm and 100 $\mu$m. According to another exemplary embodiment, the thickness of the current collector 122 is between approximately 5 $\mu$m and 25 $\mu$m. According to a particular exemplary embodiment, the thickness of the current collector 122 is approximately 10 $\mu$m.

The negative current collector 122 has a layer 124 of active material provided thereon. While FIGURE 2 shows that the layer 124 is provided on only one side of the current collector 122, it should be understood that a layer of active material similar or identical to that shown may be provided or coated on both sides of the current collector 122.

According to exemplary embodiment, the layer 124 includes a carbonaceous material such as petroleum coke, carbon fiber, hard carbon, graphite (e.g., natural graphite, synthetic graphite, potato carbon, etc.), powdered graphitized pitch (mesocarbon microbeads (MCMB)), or combinations thereof. According to an exemplary embodiment, the layer of active material provided on a negative current collector comprises between approximately 70 and 95 weight percent MCMB and between 5 and 30 weight percent coke. According to a particular exemplary embodiment, the layer of active material provided on a negative current collector comprises approximately 80 weight percent MCMB and 20 weight percent coke. According to other exemplary embodiments, the percentage of coke in the MCMB/coke layer may range between approximately 10 and 30 percent by weight. When the percentage of coke substantially exceeds the upper end of this range, negative effects such as diminished capacity and increased irreversible capacity may be exhibited by the battery, and at levels substantially below the lower end of this range, the benefits associated with the use of coke may dissipate.

A binder material may also be utilized in conjunction with the layer of active material 124 to hold the materials used in the active layer together. For example, according to an exemplary embodiment, the layer of active material may include a conductive additive such as carbon black and a binder such as polyvinylidine fluoride (PVDF) or an elastomeric polymer.

According to various exemplary embodiments, the thickness of the active material 124 is between approximately 0.1 $\mu$m and 3 mm. According to other exemplary embodiments, the thickness of the active material 124 may be between approximately 25 $\mu$m and 300 $\mu$m. According to another exemplary embodiment, the thickness of the active material 124 may be between approximately 20 $\mu$m and 90 $\mu$m, and according to a particular exemplary embodiment, may be approximately 75 $\mu$m.

An electrolyte 130 is provided intermediate or between the positive and negative electrodes to provide a medium through which lithium ions may travel. According to an exemplary embodiment, the electrolyte is a liquid and includes, for example, a lithium salt dissolved in one or more non-aqueous solvents.

According to another exemplary embodiment, the electrolyte may be a lithium salt dissolved in a polymeric material such as poly(ethylene oxide) or silicone. According to another exemplary embodiment, the electrolyte may be an ionic liquid such as N-methyl-N-alkylpyrrolidinium bis(trifluoromethanesulfohyl)imide salts. According to another exemplary embodiment, the electrolyte may be a solid state electrolyte such as a lithium-ion conducting glass such as lithium phosphorous oxynitride (LiPON).

Various other electrolytes may be used according to other exemplary embodiments. For example, according to an exemplary embodiment, the electrolyte may be a mixture of ethylene carbonate and diethyl carbonate in a 1.0 M salt of $LiPF_6$. According to another exemplary embodiment, the electrolyte may include a polypropylene carbonate solvent and a lithium bis-oxalatoborate salt (sometimes referred to as LiBOB). According to other exemplary embodiments, the electrolyte may comprise one or more of a PVDF copolymer, a PVDF-polyimide material, and organosilicon polymer, a thermal polymerization gel, a radiation cured acrylate, a particulate with polymer gel, an inorganic gel polymer electrolyte, an inorganic gel-polymer electrolyte, a PVDF gel, polyethylene oxide (PEO), a glass ceramic electrolyte, phosphate glasses, lithium conducting glasses, lithium conducting ceramics, and an inorganic ionic liquid or gel, among others.

According to an exemplary embodiment, the electrolyte includes a mixture of organic carbonates with 1 molar $LiPF_6$ as the lithium salt According to a particular exemplary embodiment, the electrolyte includes a number of organic carbonates such as propylene carbonate, ethylene carbonate, diethyl carbonate, and $LiPF_6$.

The electrolyte 130 includes two or more additives which are intended to reduce the occurrence of capacity fade in the battery 100. Such materials have been reported to aid in preventing the formation of LiF on the surface of the carbonaceous material used with negative electrodes. The formation of LiF is undesirable because it is believed that such material contributes to capacity fade and is not an advantageous component of a stable, ionically conductive layer (which may be referred to as a solid-electrolyte interphase (SEI) layer) that is formed on the negative electrode. According to an exemplary embodiment, the electrolyte 130 includes a first additive that is a borate or borane compound that acts as an anion receptor. The borate or borane compounds may include compounds containing boroxine rings (e.g., trimethoxyboroxine (TMOBX) or its derivatives or trimethyl boroxine), compounds including boroxine rings with polyalkylene oxide chains, and compounds having boroxine rings with substituted or unsubstituted phenyl rings. According to a particular exemplary embodiment, the first additive is TMOBX.

According to an exemplary embodiment, the electrolyte 130 includes a second additive that is an alkene capable of reacting (e.g., polymerizing) on the surface of the negative electrode to form an SEI layer. A particularly attractive type of such additive incorporates an unsaturated bond that is relatively easily polymerizable on the carbon surface. Alkenes which may be used for the second additive include vinylene carbonate and its derivitives, propylidene carbonate, ethylidene ethylene carbonate, isopropylidiene ethylene carbonate, vinylene acetate, vinylene ethyl carbonate, and ethyl cinnamate. According to a particular exemplary embodiment, the second additive is vinylene carbonate (VC) or one or more of its derivatives.

The inventors have determined through experimentation that an unexpected synergistic effect may be obtained by using a combination of additives. For example, additives such as VC and TMOBX have been used previously in lithium-ion batteries in the belief that these additives may contribute to the production of a more stable SEI layer. However, when an additive such as VC or TMOBX was used in the past, it was used as the only additive in the electrolyte (e.g., only VC would be added to an electrolyte in a battery). As will be illustrated in Examples 1 and 2 provided below, through experimentation, the inventors have determined that improved capacity fade performance may be achieved when a combination of additives is utilized. That is, the capacity fade will be greater in batteries that utilize only a single additive as compared to the capacity fade that may be achieved in batteries utilizing multiple additives. One possible explanation for such a result, which has not been verified experimentally, is that different additives may act in different ways to produce a more stable SEI layer, such that the use of multiple additives may provide the distinct benefits of each of the individual additives.

According to one exemplary embodiment, the electrolyte includes between approximately 0.1 and 3.0 percent TMOBX and between approximately 0.1 and 10.0 percent VC. According to one exemplary embodiment, the electrolyte includes between approximately 0.3 and 1.0 percent TMOBX and between approximately 0.1 and 10.0 percent VC. According to a particular exemplary embodiment, the electrolyte includes approximately 0.3 weight percent TMOBX and approximately 2.4 percent VC.

According to an exemplary embodiment, a lithium battery having a carbonaceous negative electrode in which the active material comprises a mixture of MCMB and coke includes an electrolyte having TMOBX and VC additives. According to another exemplary embodiment, a lithium battery having a carbonaceous negative electrode in which the active material comprises carbon fiber includes an electrolyte having TMOBX and VC additives. It is intended that the use of both TMOBX and VC additives in the electrolyte for such lithium batteries will result in batteries having better performance of the life of the batteries with a reduced tendency to exhibit significant capacity fade.

One advantageous feature of providing additives such as VC and TMOBX in the electrolyte for lithium ion batteries using carbonaceous negative electrodes is that such batteries may have longer lives with improved functionality and reliability. The voltage/capacity curves for such batteries may exhibit a more tapered shape near the end of the battery life as compared to batteries which do not utilize such additives, which may allow for more accurate elective replacement indicator (ERI) prediction. Such additives may also act to promote "leveling" reactions by which the lithium becomes more evenly distributed in the negative electrode.

EXAMPLE 1

Lithium ion cells having nominal capacities of approximately 0.3 ampere hours (Ah) were prepared having a $LiCoO_2$ positive electrode, carbonaceous negative electrode, and electrolyte that included mixtures of organic carbonates with 1 molar $LiPF_6$ as the lithium salt. The cells were constructed as spirally wound prismatic cells hermetically sealed in a stainless steel can with a glass feedthrough. The nominal capacity of the cells was approximately 0.3 ampere-hours. All cells were constructed identically, except for the differences noted below.

Twelve types of lithium ion cells were fabricated by combining three carbon types in the negative electrode, each with four electrolyte formulations. The carbon types were: (1) MCMB; (2) an MCMB/Coke Hybrid (80/20% ratio of MCMB with petroleum coke); and (3) carbon fiber. All electrodes were coated to a deposition of approximately 11 $g/cm^2$ and had a compressed density of between approximately 1.2 and 1.4 $g/cm^3$. Vendor and physical property information for these materials is provided below in Table 1.

TABLE 1

| Carbon Type | Description | Vendor | Tap Density $(g/cm^3)$ | Median Particle Size $V_{50}$ (by laser diffraction, $\mu$m) | Surface Area (BET, $m^2/g$) |
|---|---|---|---|---|---|
| MCMB | Powdered Graphitized Pitch | Osaka Gas | 1.2-1.3 | 27 | 0.45 |
| COKE | Thermocarb XP3 Needle COKE | Conoco | 0.6-0.7 | 11.4 | 5.71 |
| Carbon Fiber | Trabonic 320 | Conoco | 1.2-1.3 | 13.4 | 0.71 |

The electrolytes all had the same main combination of organic carbonates, including PC, EC, DEC, and $LiPF_6$, and differed only in the type of anti-fade additive that was used. The electrolyte types were therefore: (1) none (no additive); (2) TMOBX at 0.3 wt%; (3) VC at 2.4 wt%; and (4) a mixture of TMOBX and VC (0.3 and 2A% wt%, respectively). In electrolytes in which VC was added, the VC was added at the electrolyte vendor (EM Industries) to avoid stability problems associated with undiluted VC.

Cells were cycled on an accelerated schedule which consisted of charging at 150 mA ("C/2" rate) to 4.1 V (with 30 minute hold at 4.1 V) and discharging at 75 mA ("C/4" rate) to 2.75 V. On the first cycle, and every 100th cycle, the cells were subject to lower rate "characterization cycles." These consisted of charging at 14.5 mA and discharging at 14.5 mA, to the same voltage limits.

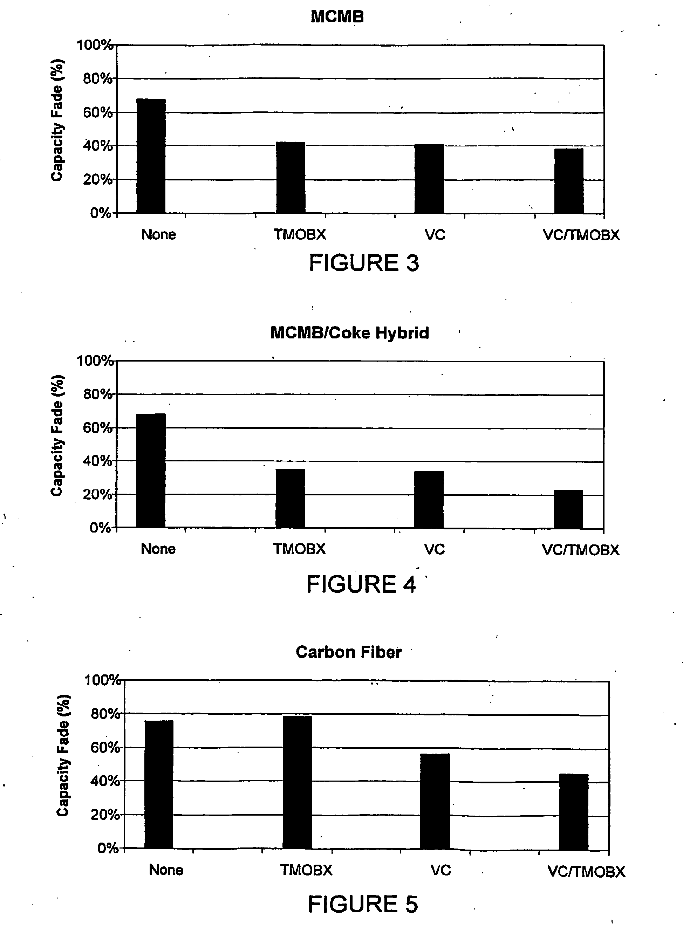

Capacity fade ($f_Q$), which is the percent capacity loss during the accelerated cycles, were determined after 1000 cycles using the formula

$$f_Q = (1 - (Q_{1001}/Q_2) * 100\%$$

where Q is discharge capacity and the numerical subscripts indicate the cycle number, Data is shown in Table 3, and graphical representations of the capacity fade results are shown in FIGURES 3-5. FIGURE 3 is a graph showing the capacity fade for lithium batteries having a negative electrode comprising MCMB and utilizing various electrolyte additives. FIGURE 4 is a graph showing the capacity fade for lithium batteries having a negative electrode comprising MCMB and coke and utilizing various electrolyte additives. FIGURE 5 is a graph showing the capacity fade for lithium batteries having a negative electrode comprising carbon fiber and utilizing various electrolyte additives.

Table 3

| Carbon Type | Electrolyte Additive | Average Capacity Fade % |
| --- | --- | --- |
| MCMB | None | 67.9 |
| | TMOBX | 42.3 |
| | VC | 41.2 |
| | VC/TMOBX | 38.2 |
| MCMB/Coke | None | 68.1 |
| | TMOBX | 35.1 |
| | VC | 34.1 |
| | VC/TMOBX | 23.0 |
| Carbon Fiber | None | 75.9 |
| | TMOBX | 78.4 |
| | VC | 56.2 |
| | VC/TMOBX | 44.9 |

It is noted that for all carbon types (MCMB, MCMB/Coke, Carbon Fiber), a reduction in capacity fade was achieved through the use of the VC/TMOBX combinations compared to either TMOBX or VC alone (i.e., while the use of VC alone, TMOBX alone, and the VC/TMOBX combination all produced measurable reductions in capacity fade as compared batteries using no additive, the VC/TMOBX additive combination produced the greatest reduction in capacity fade). As illustrated in the results shown in Table 3 and in FIGURES 3-5, where an MCMB coated negative electrode was used in the cells, the mixture of VC and TMOBX produced a relatively small reduction in capacity fade as compared to the use of TMOBX or VC alone. For the MCMB/coke hybrid electrode cells and the carbon fiber cells, however, the synergistic effect of the mixture of VC and TMOBX produced a relatively sharp decrease in capacity fade for the cells over the testing period as compared to the use of VC or TMOBX alone.

According to an exemplary embodiment, lithium-ion batteries such as those described above may be used in conjunction with any battery powered device including, but not limited to, medical devices such as medical devices that may be implanted in the human body (referred to as "implantable medical devices" or "IMD's").

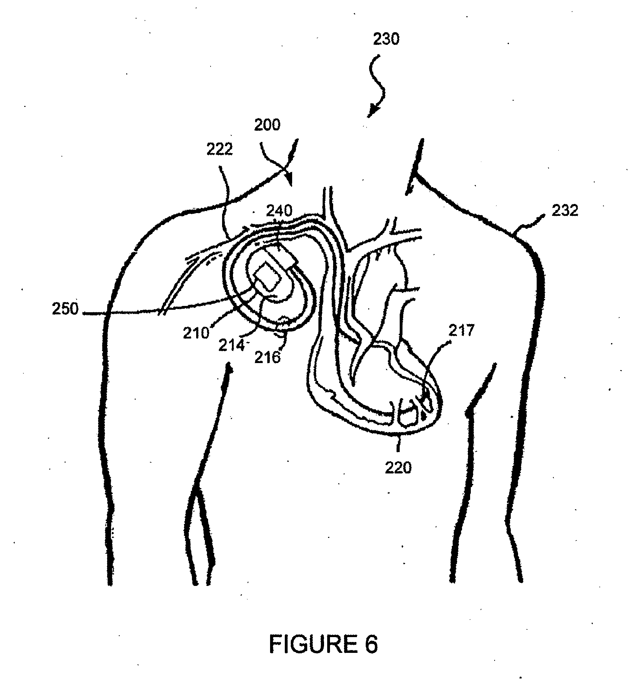

FIGURE 6 illustrates a schematic view of a system 200 (e.g., an implantable medical device) implanted within a body or torso 232 of a patient 230. The system 200 includes a device 210 in the form of an implantable medical device that for purposes of illustration is shown as a defibrillator configured to provide a therapeutic high voltage (e.g., 700 volt) treatment for the patient 230.

The device 210 includes a container or housing 214 that is hermetically sealed and biologically inert according to an exemplary embodiment. The container may be made of a conductive material. One or more leads 216 electrically connect the device 210 and to the patient's heart 220 via a vein 222. Electrodes 217 are provided to sense cardiac activity and/or provide an electrical potential to the heart 220. At least a portion of the leads 216 (e.g., an end portion of the leads shown as exposed electrodes 217) may be provided adjacent or in contact with one or more of a ventricle and an atrium of the heart 220.

The device 210 includes a battery 240 provided therein to provide power for the device 210. According to another exemplary embodiment, the battery 240 may be provided external to the device or external to the patient 230 (e.g., to allow for removal and replacement and/or charging of the battery). The size, position, and capacity of the battery 240 may be chosen based on a number of factors, including the amount of charge required for a given patient's physical or medical characteristics, the size or configuration of the device, and any of a variety of other factors. According to an exemplary embodiment, the battery is a 5 mAh battery. According to another exemplary, embodiment, the battery is a 300 mAh battery. According to various other exemplary embodiments, the battery may have a capacity of between approximately 10 and 1000 mAh. According to other exemplary embodiments, more than one battery may be provided to power the device 210. In such exemplary embodiments, the batteries may have the same capacity or one or more of the batteries may have a higher or lower capacity than the other battery or batteries.

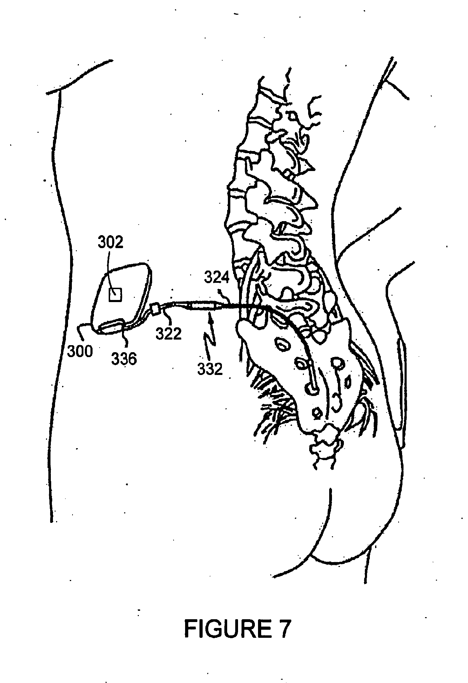

According to another exemplary embodiment shown in FIGURE 7, an implantable neurological stimulation device 300 (an implantable neuro stimulator or INS) may include a battery 302 such as those described above with respect to the various exemplary embodiments. Examples of some neuro stimulation products and related components are shown and described in a brochure titled "Implantable Neurostimulation Systems" available from Medtronic, Inc.

An INS generates one or more electrical stimulation signals that are used to influence the human nervous system or organs. Electrical contacts carried on the distal end of a lead are placed at the desired stimulation site such as the spine or brain and the proximal end of the lead is connected to the INS. The INS is then surgically implanted into an individual such as into a subcutaneous pocket in the abdomen, pectoral region, or upper buttocks area. A clinician programs the INS with a therapy using a programmer. The therapy configures parameters of the stimulation signal for the specific patient's therapy. An INS can be used to treat conditions such as pain, incontinence, movement disorders such as epilepsy and Parkinson's disease, and sleep apnea. Additional therapies appear promising to treat a variety of physiological, psychological, and emotional conditions. Before an INS is implanted to deliver a therapy, an external screener that replicates some or all of the INS functions is typically connected to the patient to evaluate the efficacy of the proposed therapy.

The INS 300 includes a lead extension 322 and a stimulation lead 324. The stimulation lead 324 is one or more insulated electrical conductors with a connector 332 on the proximal end and electrical contacts (not shown) on the distal end. Some stimulation leads are designed to be inserted into a patient percutaneously, such as the Model 3487A Pisces-Quad® lead available from Medtronic, Inc. of Minneapolis Minn., and stimulation some leads are designed to be surgically implanted, such as the Model 3998 Specify® lead also available from Medtronic.

Although the lead connector 332 can be connected directly to the INS 300 (e.g., at a point 336), typically the lead connector 332 is connected to a lead extension 322. The lead extension 322, such as a Model 7495 available from Medtronic, is then connected to the INS 300.

Implantation of an INS 320 typically begins with implantation of at least one stimulation lead 324, usually while the patient is under a local anesthetic. The stimulation lead 324 can either be percutaneously or surgically implanted. Once the stimulation lead 324 has been implanted and positioned, the stimulation lead's 324 distal end is typically anchored into position to minimize movement of the stimulation lead 324 after implantation. The stimulation lead's 324 proximal end can be configured to connect to a lead extension 322.

The INS 300 is programmed with a therapy and the therapy is often modified to optimize the therapy for the patient (i.e., the INS may be programmed with a plurality of programs or therapies such that an appropriate therapy may be administered in a given situation). In the event that the battery 302 requires recharging, an external lead (not shown) may be used to electrically couple the battery to a charging device or apparatus.

A physician programmer and a patient programmer (not shown) may also be provided to allow a physician or a patient to control the administration of various therapies. A physician programmer, also known as a console programmer, uses telemetry to communicate with the implanted INS 300, so a clinician can program and manage a patient's therapy stored in the INS 300, troubleshoot the patient's INS 300 system, and/or collect data. An example of a physician programmer is a Model 7432 Console Programmer available from Medtronic. A patient programmer also uses telemetry to communicate with the INS 300, so the patient can manage some aspects of her therapy as defined by the clinician. An example of a patient programmer is a Model 7434 Itrel® 3 EZ Patient Programmer available from Medtronic.

While the medical devices described herein (e.g., systems 200 and 300) are shown and described as a defibrillator and a neurological stimulation device, it should be appreciated that other types of implantable medical devices may be utilized according to other exemplary embodiments, such as pacemakers, cardioverters, cardiac contractility modulators, drug administering devices, diagnostic recorders, cochlear implants, and the like for alleviating the adverse effects of various health ailments. According to still other embodiments, non-implantable medical devices or other types of devices may utilize batteries as are shown and described in this disclosure.

It is also contemplated that the medical devices described herein may be charged or recharged when the medical device is implanted within a patient. That is, according to an exemplary embodiment, there is no need to disconnect or remove the medical device from the patient in order to charge or recharge the medical device. For example, transcutaneous energy transfer (TET) may be used, in which magnetic induction is used to deliver energy from outside the body to the implanted battery, without the need to make direct physical contact to the implanted battery, and without the need for any portion of the implant to protrude from the patient's skin. According to another exemplary embodiment, a connector may be provided external to the patient's body that may be electrically coupled to a charging device in order to charge or recharge the battery. According to other exemplary embodiments, medical devices may be provided that may require removal or detachment from the patient in order to charge or recharge the battery.

It is important to note that the construction and arrangement of the lithium-ion battery as shown and described with respect to the various exemplary embodiments is illustrative only. Although only a few embodiments of the present inventions have been described in detail in this disclosure, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter recites in the claims. Accordingly, all such modifications are intended to be included within the scope of the present invention as defined in the appended claims. Other substitutions, modifications, changes and omissions may be made in the design, operating conditions and arrangement of the preferred and other exemplary embodiments without departing from the scope of the present invention as expressed in the appended claims.

**Claims**

1. A lithium-ion battery (100) comprising:

    a positive electrode (110);
    a negative electrode (120) comprising carbon; and
    an electrolyte (130) containing a first and second additive;
    wherein the first additive comprises a compound comprising at least one boroxine ring that acts as an ion receptor and the second additive comprises an alkene capable of reacting on a surface of the negative electrode (120) to form an ionically conductive layer, the alkene selected from the group consisting of vinylene carbonate and derivatives thereof, propylidene carbonate, ethylidene ethylene carbonate, isopropylidiene ethylene carbonate, vinylene acetate, vinylene ethyl carbonate, and ethyl cinnamate.

2. The lithium-ion battery (100) according to claim 1, wherein the first additive comprises trimethoxyboroxine or a derivative thereof.

3. The lithium-ion battery (100) according to claim 1, wherein the first additive comprises at least one material selected from the group consisting of trimethyl boroxine, compounds including boroxine rings with polyalkylene oxide chains, and compounds having boroxine rings with substituted or unsubstituted phenyl rings.

4. The lithium-ion battery (100) according to claim 2 or 3, wherein the second additive comprises vinylene carbonate or a derivative thereof.

5. The lithium-ion battery (100) according to claim 2 or 3, wherein the second additive comprises at least one material selected from the group consisting of propylidene carbonate, ethylidene ethylene carbonate, isopropylidiene ethylene carbonate, vinylene acetate, vinylene ethyl carbonate, and ethyl cinnamate.

6. The lithium-ion battery (100) according to any of the preceding claims, wherein the first additive is present in the electrolyte (130) in an amount between approximately 0.1 and 3.0 percent and the second additive is present in an amount between approximately 0.1 and 10.0 percent.

7. The lithium-ion battery (100) according to any of the preceding claims, wherein the electrolyte (130) comprises approximately 0.3 weight percent of the first additive and approximately 2.4 percent of the second additive.

8. The lithium-ion battery (100) according to any of the preceding claims, wherein the carbon of the negative electrode (120) comprises a material selected from the group consisting of coke, carbon fiber, hard carbon, graphite, graphitized mesocarbon microbeads, and combinations thereof.

9. The lithium-ion battery (100) according to any of the preceding claims, wherein the carbon of the negative electrode (120) comprises a mixture of graphitic and non-graphitic carbon.

10. The lithium-ion battery (100) according to claim 9, wherein the graphitic carbon comprises mesocarbon microbeads and the non-graphitic carbon comprises coke.

11. The lithium-ion battery (100) according to claim 10, wherein the mesocarbon microbeads and coke are provided in a ratio of approximately 4:1 by weight.

12. The lithium-ion battery (100) according to any of claims 1 through 10, wherein the carbon of the negative electrode (120) comprises between approximately 70 and 95 weight percent mesocarbon microbeads and between approximately 5 and 30 weight percent coke.

13. The lithium-ion battery (100) according to any of claims 1 through 10, wherein the carbon of the negative electrode (120) comprises approximately 80 weight percent mesocarbon microbeads and approximately 20 weight percent coke.

14. The lithium-ion battery (100) according to claim 1, wherein the carbon of the negative electrode (120) comprises mesocarbon microbeads and coke, the first additive is trimethoxyboroxine or a derivative thereof provided in the electrolyte (130) at approximately 0.3 weight percent, and the second additive is vinylene carbonate or a derivative thereof provided in the electrolyte (130) at approximately 2.4 weight percent.

15. The lithium-ion battery (100) according to claim 1, wherein the carbon of the negative electrode (120) comprises carbon fibers, the first additive is trimethoxyboroxine or a derivative thereof, and the second additive is vinylene carbonate or a derivative thereof.

16. The lithium-ion battery (100) according to claim 15, wherein the electrolyte (130) comprises approximately 0.3 weight percent of the first additive and approximately 2.4 weight percent of the second additive.

17. A medical device comprising a lithium-ion battery (100) according to any of the preceding claims.

18. The medical device according to Claim 17, wherein at least a portion of the medical device is configured for implantation into a body of a patient and the lithium-ion battery (100) may be charged without removing the battery from the body of the patient.

19. The medical device according to Claim 17 or 18, wherein the medical device comprises a neurological stimulation device.

20. The medical device according to Claim 17 or 18, wherein the medical device is selected from the group consisting of a cardiac defibrillator, a cardiac pacemaker, a cardiac contractility modulator, a cardioverter, a drug administration device, a cochlear implant, a hearing aid, a sensor, a telemetry device, and a diagnostic recorder.

**Patentansprüche**

1. Lithiumionenbatterie (100), umfassend:

    eine positive Elektrode (110);
    eine negative Elektrode (120), umfassend Kohlenstoff; und
    einen Elektrolyt (130), enthaltend ein erstes und zweites Additiv;
    wobei das erste Additiv eine Verbindung umfasst, die mindestens einen Boroxinring umfasst, der als ein Ionenrezeptor wirkt, und das zweite Additiv ein Alken umfasst, das auf einer Oberfläche der negativen Elektrode (120) reagieren kann, um eine ionisch leitende Schicht zu bilden, das Alken ausgewählt ist aus der Gruppe, bestehend aus Vinylencarbonat und Derivaten davon, Propylidencarbonat, Ethylidenethylencarbonat, Isopropylidenethylencarbonat, Vinylenacetat, Vinylenethylcarbonat und Ethylcinnamat.

2. Lithiumionenbatterie (100) nach Anspruch 1, wobei das erste Additiv Trimethoxyboroxin oder ein Derivat davon umfasst.

3. Lithiumionenbatterie (100) nach Anspruch 1, wobei das erste Additiv mindestens ein Material umfasst, ausgewählt aus der Gruppe, bestehend aus Trimethylboroxin, Verbindungen, einschließend Boroxinringe mit Polyalkylenoxidketten und Verbindungen mit Boroxinringen mit substituierten oder unsubstituierten Phenylringen.

4. Lithiumionenbatterie (100) nach Anspruch 2 oder 3, wobei das zweite Additiv Vinylencarbonat oder ein Derivat davon umfasst.

5. Lithiumionenbatterie (100) nach Anspruch 2 oder 3, wobei das zweite Additiv mindestens ein Material umfasst, ausgewählt aus der Gruppe, bestehend aus Propylidencarbonat, Ethylidenethylencarbonat, Isopropylidenethylencarbonat, Vinylenacetat, Vinylenethylcarbonat und Ethylcinnamat.

6. Lithiumionenbatterie (100) nach einem der vorangehenden Ansprüche, wobei das erste Additiv im Elektrolyt (130) in einer Menge zwischen etwa 0,1 und 3,0 Prozent vorliegt und das zweite Additiv in einer Menge zwischen etwa 0,1 und 10,0 Prozent vorliegt.

7. Lithiumionenbatterie (100) nach einem der vorangehenden Ansprüche, wobei der Elektrolyt (130) etwa 0,3 Gew.-

% des ersten Additivs und etwa 2,4 Prozent des zweiten Additivs umfasst.

**8.** Lithiumionenbatterie (100) nach einem der vorangehenden Ansprüche, wobei der Kohlenstoff der negativen Elektrode (120) ein Material umfasst, ausgewählt aus der Gruppe, bestehend aus Koks, Kohlefaser, hartem Kohlenstoff, Graphit, graphitisierten Mesocarbonmikrokügelchen und Kombinationen davon.

**9.** Lithiumionenbatterie (100) nach einem der vorangehenden Ansprüche, wobei der Kohlenstoff der negativen Elektrode (120) ein Gemisch aus graphitischem und nicht-graphitischem Kohlenstoff umfasst.

**10.** Lithiumionenbatterie (100) nach Anspruch 9, wobei der graphitische Kohlenstoff Mesocarbonmikrokügelchen und der nicht-graphitische Kohlenstoff Koks umfasst.

**11.** Lithiumionenbatterie (100) nach Anspruch 10, wobei die Mesocarbonmikrokügelchen und der Koks in einem Gewichtsverhältnis von etwa 4:1 bereitgestellt sind.

**12.** Lithiumionenbatterie (100) nach einem der Ansprüche 1 bis 10, wobei der Kohlenstoff der negativen Elektrode (120) zwischen etwa 70 und 95 Gew.-% Mesocarbonmikrokügelchen und zwischen etwa 5 und 30 Gew.-% Koks umfasst.

**13.** Lithiumionenbatterie (100) nach einem der Ansprüche 1 bis 10, wobei der Kohlenstoff der negativen Elektrode (120) etwa 80 Gew.-% Mesocarbonmikrokügelchen und etwa 20 Gew.-% Koks umfasst.

**14.** Lithiumionenbatterie (100) nach Anspruch 1, wobei der Kohlenstoff der negativen Elektrode (120) Mesocarbonmikrokügelchen und Koks umfasst, das erste Additiv Trimethoxyboroxin oder ein Derivat davon ist, das in dem Elektrolyt (130) mit etwa 0,3 Gew.-% bereitgestellt ist, und das zweite Additiv Vinylencarbonat oder ein Derivat davon ist, das in dem Elektrolyt (130) mit etwa 2,4 Gew.-% bereitgestellt ist.

**15.** Lithiumionenbatterie (100) nach Anspruch 1, wobei der Kohlenstoff der negativen Elektrode (120) Kohlefasern umfasst, das erste Additiv Trimethoxyboroxin oder ein Derivat davon ist und das zweite Additiv Vinylencarbonat oder ein Derivat davon ist.

**16.** Lithiumionenbatterie (100) nach Anspruch 15, wobei der Elektrolyt (130) etwa 0,3 Gew.-% des ersten Additivs und etwa 2,4 Gew.-% des zweiten Additivs umfasst.

**17.** Medizinische Vorrichtung, umfassend eine Lithiumionenbatterie (100) nach einem der vorangehenden Ansprüche.

**18.** Medizinische Vorrichtung nach Anspruch 17, wobei mindestens ein Teil der medizinischen Vorrichtung zur Implantation in einen Körper eines Patienten konfiguriert ist und die Lithiumionenbatterie (100) geladen werden kann, ohne die Batterie aus dem Körper des Patienten zu entfernen.

**19.** Medizinische Vorrichtung nach Anspruch 17 oder 18, wobei die medizinische Vorrichtung eine neurologische Stimulationsvorrichtung umfasst.

**20.** Medizinische Vorrichtung nach Anspruch 17 oder 18, wobei die medizinische Vorrichtung ausgewählt ist aus der Gruppe, bestehend aus einem Herzdefibrillator, einem Herzschrittmacher, einem Herzkontraktionsmodulator, einem Defibrillator, einer Arzneistoffabgabevorrichtung, einem Innenohrimplantat, einem Hörgerät, einem Sensor, einer Telemetrievorrichtung und einem Diagnoseaufzeichnungsgerät.

## Revendications

**1.** Batterie au lithium-ion (100) comprenant : une électrode positive (110) ;
une électrode négative (120) comprenant du carbone ; et un électrolyte (130) contenant des premier et second additifs ;
dans lequel le premier additif comprend un composé comprenant au moins un noyau boroxine qui agit comme récepteur d'ions et le second additif comprend un alcène capable de réagir sur une surface de l'électrode négative (120) afin de former une couche conductrice par migration des ions, l'alcène étant choisi dans le groupe constitué par le carbonate de vinylène et ses dérivés, le carbonate de propylidène, le carbonate d'éthylidène éthylène, le carbonate d'isopropylidène éthylène, l'acétate de vinylène, l'éthylcarbonate de vinylène et le cinnamate d'éthyle.

**2.** Batterie au lithium-ion (100) selon la revendication 1, dans laquelle le premier additif comprend de la triméthoxyboroxine ou un dérivé de celle-ci.

**3.** Batterie au lithium-ion (100) selon la revendication 1, dans laquelle le premier additif comprend au moins une matière choisie dans le groupe constitué par la triméthyl boroxine, des composés comprenant des noyaux boroxine avec des chaînes poly(oxyde d'alkylène) et des composés ayant des noyaux boroxine avec des noyaux phényle substitués ou non-substitués.

**4.** Batterie au lithium-ion (100) selon l'une des revendications 2 ou 3, dans laquelle le second additif comprend du carbonate de vinylène ou un dérivé de celui-ci.

**5.** Batterie au lithium-ion (100) selon l'une des revendications 2 ou 3, dans laquelle le second additif comprend au moins une matière choisie dans le groupe constitué par le carbonate de propylidène, le carbonate d'éthylidène éthylène, le carbonate d'isopropylidène éthylène, l'acétate de vinylène, l'éthylcarbonate de vinylène et le cinnamate d'éthyle.

**6.** Batterie au lithium-ion (100) selon l'une quelconque des revendications précédentes, dans laquelle le premier additif est présent dans l'électrolyte (130) dans une quantité entre approximativement 0,1 et 3,0 pour cent et le second additif est présent dans une quantité entre approximativement 0,1 et 10,0 pour cent.

**7.** Batterie au lithium-ion (100) selon l'une quelconque des revendications précédentes, dans laquelle l'électrolyte (130) comprend approximativement 0,3 pour cent en poids du premier additif et approximativement 2,4 pour cent du second additif.

**8.** Batterie au lithium-ion (100) selon l'une quelconque des revendications précédentes, dans laquelle le carbone de l'électrode négative (120) comprend une matière choisie dans le groupe constitué par le coke, la fibre de carbone, le carbone dur, le graphite, les microbilles de mésocarbone graphités et leurs combinaisons.

**9.** Batterie au lithium-ion (100) selon l'une quelconque des revendications précédentes, dans laquelle le carbone de l'électrode négative (120) comprend un mélange de carbone graphitique et non-graphitique.

**10.** Batterie au lithium-ion (100) selon la revendication 9, dans laquelle le carbone graphitique comprend des microbilles de mésocarbone et le carbone non-graphitique comprend du coke.

**11.** Batterie au lithium-ion (100) selon la revendication 10, dans laquelle les microbilles de mésocarbone et le coke sont fournis dans un rapport d'approximativement 4:1 en poids.

**12.** Batterie au lithium-ion (100) selon l'une quelconque des revendications 1 à 10, dans laquelle le carbone de l'électrode négative (120) comprend entre approximativement 70 et 95 pour cent en poids de microbilles de mésocarbone et entre approximativement 5 et 30 pour cent en poids de coke.

**13.** Batterie au lithium-ion (100) selon l'une quelconque des revendications 1 à 10, dans laquelle le carbone de l'électrode négative (120) comprend approximativement 80 pour cent en poids de microbilles de mésocarbone et approximativement 20 pour cent en poids de coke.

**14.** Batterie au lithium-ion (100) selon la revendication 1, dans laquelle le carbone de l'électrode négative (120) comprend des microbilles de mésocarbone et du coke, le premier additif est la triméthoxyboroxine ou un dérivé de celle-ci disposé dans l'électrolyte (130) à approximativement 0,3 pour cent en poids, et le second additif est le carbonate de vinylène ou un dérivé de celui-ci disposé dans l'électrolyte (130) à approximativement 2,4 pour cent en poids.

**15.** Batterie au lithium-ion (100) selon la revendication 1, dans laquelle le carbone de l'électrode négative (120) comprend des fibres de carbone, le premier additif est la triméthoxyboroxine ou un dérivé de celle-ci, et le second additif est le carbonate de vinylène ou un dérivé de celui-ci.

**16.** Batterie au lithium-ion (100) selon la revendication 15, dans laquelle l'électrolyte (130) comprend approximativement 0,3 pour cent en poids du premier additif et approximativement 2,4 pour cent en poids du second additif.

**17.** Dispositif médical comprenant une batterie au lithium-ion (100) selon l'une quelconque des revendications précédentes.

**18.** Dispositif médical selon la revendication 17, dans lequel au moins une partie du dispositif médical est configurée pour une implantation dans un corps d'un patient et la batterie au lithium-ion (100) peut être chargée sans retirer la batterie du corps du patient.

**19.** Dispositif médical selon la revendication 17 ou 18, dans lequel le dispositif médical comprend un dispositif de stimulation neurologique.

**20.** Dispositif médical selon la revendication 17 ou 18, dans lequel le dispositif médical est choisi dans le groupe constitué par un défibrillateur cardiaque, un stimulateur électrique cardiaque, un modulateur de contractilité cardiaque, un défibrillateur à synchronisation automatique, un dispositif d'administration de médicament, un implant cochléaire, une prothèse auditive, un détecteur, un dispositif de télémétrie et un enregistreur de diagnostic.

( - )

34

32

30

CHARGE

DISCHARGE

40

( + )

24

22

20

10

V

FIGURE 1

EP 1 932 203 B1

FIGURE 2

EP 1 932 203 B1

## MCMB

FIGURE 3

## MCMB/Coke Hybrid

FIGURE 4

## Carbon Fiber

FIGURE 5

FIGURE 6

EP 1 932 203 B1

FIGURE 7

EP 1 932 203 B1

**EP 1 932 203 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 97904104 A